(19) Europäisches Patentamt
European Patent Office
Office européen des brevets

(11) Veröffentlichungsnummer: **0 253 254 B1**

# (12) EUROPÄISCHE PATENTSCHRIFT

(45) Veröffentlichungstag der Patentschrift: **11.09.91**

(21) Anmeldenummer: **87109679.8**

(22) Anmeldetag: **06.07.87**

(51) Int. Cl.5: **G01N 33/546**, C08F 220/52, C08F 228/00, C08F 226/00, C08F 291/00

## (54) Dispersionspolymere, biologisch aktive Dispersionspolymere, Verfahren zu ihrer Herstellung und Verwendung als diagnostisches Mittel.

(30) Priorität: **09.07.86 DE 3622993**

(43) Veröffentlichungstag der Anmeldung:
**20.01.88 Patentblatt 88/03**

(45) Bekanntmachung des Hinweises auf die Patenterteilung:
**11.09.91 Patentblatt 91/37**

(84) Benannte Vertragsstaaten:
**AT BE CH DE ES FR GB IT LI LU NL SE**

(56) Entgegenhaltungen:
**EP-A- 0 080 614**
**GB-A- 1 469 358**
**US-A- 4 547 463**

(73) Patentinhaber: **BEHRINGWERKE Aktiengesellschaft**
**Postfach 1140**
**W-3550 Marburg 1(DE)**

(72) Erfinder: **Dengler, Michael, Dr.**
**Unter den Eichen 23**
**W-3550 Marburg(DE)**
Erfinder: **Kapmeyer, Wolfgang, Dr.**
**Reinhardswaldstrasse 5**
**W-3550 Marburg(DE)**
Erfinder: **Rinno, Helmut, Dr.**
**Goethestrasse 38**
**W-6238 Hofheim am Taunus(DE)**

(74) Vertreter: **Klein, Otto, Dr. et al**
**Hoechst AG Zentrale Patentabteilung Postfach 80 03 20**
**W-6230 Frankfurt am Main 80(DE)**

EP 0 253 254 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Dispersionspolymere, Verfahren zu ihrer Herstellung und ihre Verwendung, wobei die Dispersionspolymere aus Latexpartikeln bestehen, auf deren äußerer Schicht sich ein Copolymerisat aus Vinylmonomeren befindet, von denen eines eine N-Alkylbetain-Acrylamid-Verbindung oder ein N-Alkylbetain-Acrylsäureester bzw. ein entsprechendes Derivat mit Betainstruktur der Methacrylsäure ist. Man gewinnt daraus biologisch aktive Dispersionspolymere, indem man niedermolekulare oder hochmolekulare biologisch aktive Substanzen, die über freie Aminogruppen verfügen, an reaktive, von der Aldehydfunktion abgeleitete Gruppen auf der Oberfläche des erfindungsgemäßen Latex bindet. Diese biologisch aktiven Dispersionspolymere sind vor allem für serologische oder immunologische Bestimmungsmethoden geeignet.

Es ist bekannt, daß die Empfindlichkeit serologischer oder immunologischer Bestimmungsverfahren durch Verwendung von Trägerpartikeln, die mit den entsprechenden immunologischen Reagenzien beladen sind, erhöht werden kann. Als biologisches Trägermaterial können z.B. rote Blutkörperchen oder Zellen einer Zellkultur verwendet werden. Zum gleichen Zweck werden auch Latexpartikel mit einem Durchmesser von 0,02-5 $\mu$m verwendet.

Ferner ist bekannt, daß bei Polymerlatices mit Kern-Schale-Aufbau sich bevorzugt hydrophile Komponenten, die keine stark ionischen Eigenschaften besitzen, zur kovalenten Immobilisierung von biologisch wirksamen Substan zen im Hinblick auf immunologische Tests eignen (EP-A 65 069).

Aus der Europäischen Patentanmeldung EP-A 80 614 (im folgenden als Stand der Technik bezeichnet) sind Latexpartikel bekannt, die über Säureamidgruppen gebundene Acetal-Funktionen enthalten. In einem wässrigen Medium vorgefertige Latexkerne werden mit Vinylmonomeren, die über Säureamidgruppen gebundene Acetal-Funktionen enthalten, angequollen und diese Vinylmonomere, die ausreichend wasserunlöslich sein müssen, werden dann zusammen mit weiteren Monomeren, die hydrophiler oder ionischer Natur sein können, copolymerisiert. Derartige Reagenzien lassen sich beispielsweise zur nephelometrischen und turbidimetrischen Bestimmung von C-reaktivem Protein einsetzen. Dazu werden Serumproben hoch mit Puffer verdünnt, normalerweise 1:100, wodurch störende Serumproteine, die anderfalls zu falschpositiven oder falschnegativen Ergebnissen führen würden, vernachlässigbar werden.

Diese Arbeitsweise ist im allgemeinen dann möglich, wenn für die diagnostischen Untersuchungen Proben vorliegen, bei denen die Konzentrationen des Analyten, also z.B. von C-reaktivem Protein, mehr als 5 mg/l betragen. Will man aber die Konzentration von Spurenproteinen im Bereich von 1 $\mu$g/l bis 50 $\mu$g/l messen, dann dürfen die Proben nicht entsprechend hoch mit Puffer verdünnt werden, weil sonst die Konzentration des nachzuweisenden Proteins so gering wird, daß die Nachweisempfindlichkeit nicht ausreicht.

Eine Steigerung der Nachweisempfindlichkeit bei Latexpräparationen nach dem Stand der Technik ist jedoch nicht ohne weiteres möglich und liefert zum Beispiel für die Bestimmung von Myoglobin und anderen Substanzen keine zufriedenstellend funktionierenden Tests.

Der Versuch, die Empfindlickeit zu steigern, führt dazu, daß die Signale für die Messung einer Referenzkurve schon nach relativ kurzer Zeit unspezifisch derart ansteigen, daß eine Auswertung nicht mehr möglich ist. Die Ursache liegt darin, daß die einzelnen Partikel eines derartigen, instabilen Reagenzes sich ohne die Anwesenheit des Analyten agglutinierend zusammenballen und so zu einer Erhöhung des Streulichtsignales oder der Extinktion führen.

Es wurde nun überraschend gefunden, daß die geschilderten Nachteile des Standes der Technik überwunden werden können, wenn man Trägerpartikel verwendet, die dadurch hergestellt sind, daß vorgefertigte Latexkerne in einem wässrigen Medium mit Acryl- oder Methacrylmonomeren, die über Säureamidgruppen gebundene Acetal-Funktionen enthalten, zusammen mit Acryl- oder Methacrylmonomeren, die ein betainartiges Strukturelement tragen, copolymerisiert werden.

Gegenstand der Erfindung sind somit Dispersionspolymere, die Trägerteilchen enthalten, welche aus Latex teilchen in einem wässrigen Medium bestehen auf deren Oberfläche sich ein Copolymerisat befindet, das Monomeren mit endständigen Acetalen der Formel I

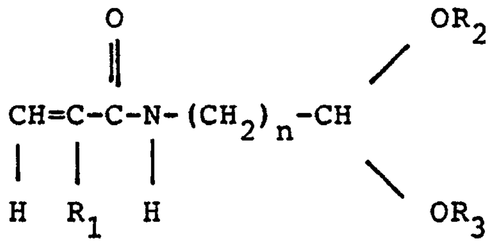

Formel I

worin
n = 1-6;
$R_1$ = H, $CH_3$ und
$R_2$ und $R_3$ gleich oder unterschiedlich sind mit
$R_2$, $R_3$ = $-(CH_2)_m-CH_3$ m = 0-7 oder

$R_2$, $R_3$ = 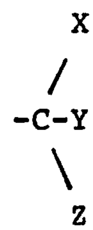 ,

wobei X, Y, Z = $(CH_2)_p-CH_3$ und P = 1-3 sind,
mit X, Y, Z gleich oder unterschiedlich
und Monomeren der Formel II

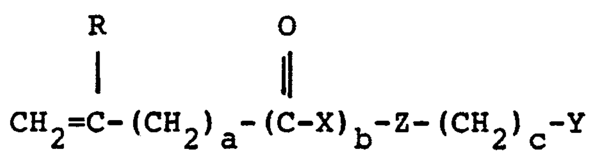

Formel II

wobei
R = H, $CH_3$
X = -NH, -O
Y = $SO_3$ , $COO^-$

$$Z = -(CH_2)_k-{}^{+}N\left[(CH_2)_m-H\right]_2-, \quad \text{mit } k = 1\text{-}12, \quad m = 1\text{-}6$$

oder

Z = 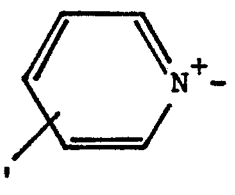 mit R' = H, 1-3 C-alkyl

a = 0- 6
b = 0- 1
c = 1-12
bedeuten enthält.

Die erfindungsgemäßen Dispersionspolymere (bzw. Latices) können durch Copolymerisation auf üblichen, herkömmlichen Latexpartikeln als Saatdispersion hergestellt werden, die mittels bekannter Verfahren als Homo- oder Copolymerisate von Monomeren erhalten werden können.

Bevorzugt werden Dispersionspolymere gemäss Anspruch 2.

Die Latex-Partikel, die als Saatdispersion für die erfindungsgemäßen Dispersionen eingesetzt werden, sollen nicht-filmbildende Polymerisate sein. Unter "nicht-filmbildend" werden Polymer-Latexteilchen verstanden, die keinen Film unter den hier in Frage kommenden Anwendungsbedingungen bilden und nicht zusammenfließen. Polymerisate aus carbocyclischen aromatischen Monovinylidenmonomeren, wie Styrol, Vinyltoluol und Vinylnaphthalin sowie Mischungen dieser Monomeren untereinander und/oder mit Methylmethacrylat und Acrylnitril sind bevorzugt. Besonders bevorzugte Saatdispersionen sind Polystyrol-Latices.

Zur Herstellung der erfindungsgemäßen Dispersionspolymere wird grundsätzlich ein vorgefertigter Latex mit Partikeldurchmesser von 0,02 bis 2 µm, vorzugsweise 0,05 bis 0,5 µm, mit etwa 20-80 % der Menge eines Emulgators versetzt, die zur maximalen monomolekularen Bedeckung der Latexoberfläche notwendig wäre.

Messungen zur Bestimmung der Emulgatormenge, die zu einer maximalen Bedeckung der Latexoberfläche führt, werden mit Hilfe eines Tensiometers durchgeführt. Sie sind zum Beispiel von I. Phrma und S.-R. Chen im Journal of Colloid and Interface Science, Vol. 74 (1979), S. 90-102 und erstmalig von S.H. Maron, M.E. Elder und I.N. Ulevitch im Journal of Colloid Interface Science, Vol. 9 (1954), S. 89-104, publiziert worden.

In Frage kommende Emulgatoren sind beispielsweise Polyglycolether mit langkettigen, aliphatischen Alkoholen, die vorzugsweise 10-20 Kohlenstoffatome aufweisen, oder Alkylphenole, deren Alkylrest vorzugsweise 6-12 Kohlenstoffatome enthält, oder Dialkylphenole oder Trialkylphenole , deren Alkylreste vorzugsweise verzweigte Alkylreste mit jeweils 3-10 Kohlenstoffatomen darstellen. Beispiele hierfür sind Umsetzungsprodukte von Ethylenoxyd mit Laurylalkohol, Stearylalkohol, Oleylalkohol, Kokosfettalkohol, Octylphenol, Nonylphenol, Di-iscpropylphenol, Tri-isopropylphenol, Di-t-butylphenol und Tri-t-butylphenol. Reaktionsprodukte des Ethylenoxids mit Polypropylenglykol oder Polybutylenglykol sind ebenfalls geeignet.

Von den ionischen Emulgatoren sind vor allem anionische Emulgatoren geeignet, insbesondere Alkalioder Ammoniumsalze von Alkylsulfonaten, Arylsulfaten oder Alkylarylsulfonaten sowie von den entsprechenden Sulfaten, Phosphaten oder Phosphonaten, die gegebenenfalls Oxyethylen-Einheiten zwischen dem jeweiligen Kohlenwasserstoffrest und anionischen Gruppe aufweisen. Beispiele hierfür sind Natriumdodecylsulfat, Natriumlaurylsulfat, Natriumoctylphenolglykolethersulfat, Natriumdodecylbenzolsulfonat, Natriumlauryldiglykolsulfat, Ammonium-tri-t-butylphenolpentaglykolsulfat und Ammonium-tri-t-butylphenoloctaglykolsulfat. Bevorzugt eingesetzt wird Natriumdodecylsulfat.

Die Polymerisation erfolgt nach an sich bekannten Verfahren in Gegenwart eines radikalbildenden Initiators, z.B. einer Peroxid-Verbindung oder einer aliphatischen Azoverbindung.

Der einzusetzende Initiator ist vorzugsweise wasserlöslich; er wird in einer Menge von 0,05 bis 10 Gew. %, vorzugsweise 0,1 bis 3 Gew. % eingesetzt (bezogen auf die Gesamtmenge der Mo nomeren). Bekannte radikalbildende Initiatoren sind zum Beispiel Wasserstoffperoxid, Alkalioder Ammoniumsalze der Peroxydischwefelsäure oder der Peroxydiphosphorsäure, zum Beispiel Natriumperoxydisulfat, Kaliumperoxydisulfat

und Ammoniumperoxydisulfat, ferner Alkylhydroperoxide wie t-Butylhydroperoxid, Dialkylperoxide wie Di-t-butylperoxid, Diacylperoxide wie Diacetylperoxid, Dilauroylperoxid und Dibenzoylperoxid sowie Azodiisobuttersäurenitril, Azodicarbonamid und Azo-gamma,gamma'-bis(4-cyanvaleriansäure). Bevorzugt eingesetzt werden die Alkali- oder Ammoniumsalze der Peroxydischwefelsäure, wie Natrium-, Kalium- und Ammoniumperoxydisulfat.

Der Initiator wird gegebenenfalls zusammen mit einem Reduktionsmittel eingesetzt, insbesondere mit einem Alkalisalz oder Erdalkalisalz einer reduzierend wirkenden schwefelhaltigen Säure; vorzugsweise eignen sich Sulfite, Bisulfite, Pyrosulfite, Dithionite, Thiosulfate und Formaldehydsulfoxylate. Gleichfalls verwendbar sind auch Glukose und Ascorbinsäure.

Zu der Saatdispersion, die Emulgator und Radikalstarter enthält, wird die Monomerenmischung aus Betainstrukturgruppen enthaltendem Monomer der Formel II und Acetalgruppen enthaltendem Monomer der Formel I sowie gegebenenfalls weitere Komponenten wie Styrol, Vinylnaphthalin oder Vinyltoluol und Methacrylsäure, Acrylsäure oder Crotonsäure unter Rühren zugetropft. Die Temperatur der Dispersion liegt zwischen + 10° und + 120° C, vorzugsweise zwischen + 50° und + 90° C.

Als Betainstrukturgruppen enthaltendes Monomer eignen sich die Betaine der Formel II, wie z.B. das 1-(3-Sulfopropyl)-2-vinyl-pyridiniumbetain. Bevorzugt sind Verbindungen gemäss Anspruch 3. Vorzugsweise werden die N-(3-Sulfopropyl)-N,N-dialkyl-N-alkylacryl-oder methacrylverbindungen eingesetzt.

Besonders bevorzugt sind N-(3-Sulfopropyl)-N-methacryloxyethyl-N,N-dimethylammoniumbetain und N-(3-Sulfopropyl)-N-methacrylamidopropyl-N,N-dimethylammoniumbetain.

Als Acetalgruppen enthaltende Monomere werden die Verbindungen der Formel I eingesetzt, bevorzugt verwendet man Acryl-oder Methacrylamidoalkylaldehyd-di-alkylacetal mit Alkyl = $C_1$ bis $C_8$. Ganz besonders geeignet sind Acryl- oder Methacrylamidoacetaldehyd-di-n-pentylacetal.

Die Monomeren gemäß Formel I und II werden als Mischung der Saatdispersion zugesetzt, wobei die Monomerenmischung aus dem Betain der Formel II in Mengen von 10 bis 90 Gew. %, vorzugsweise 25 bis 70 Gew. % und aus der Acetalverbindung nach Formel I in Mengen von 90 bis 10 Gew. %, vorzugsweise 70 bis 25 Gew. % besteht.

Dem Monomerengemisch können bis zu 50 Gew. %, bezogen auf die Gesamtmischung, Styrol, Vinylnaphthalin oder Vinyltoluol zugegeben werden. Außerdem kann das Monomerengemisch, gegebenenfalls zusätzlich, bis zu 30 Gew. %, bezogen auf die Gesamtmischung, Methacrylsäure, Acrylsäure oder Crotonsäure enthalten.

Vorteilhafterweise setzt man der aus den Monomeren bestehenden Mischung bis zu 90 Gew. %, bezogen auf die Gesamtmischung, Dimethylformamid oder andere geeignete Substanzen zu, die die Viskosität herabsetzen. Besonders vorteilhaft ist es, aus dem Monomerengemisch durch Zusatz einer wässrigen Emulgatorlösung eine Emulsion zu bilden. Dabei können bis zu 90 Gew. %, bezogen auf die Gesamtmischung, der wässrigen Emulgatorlösung, die bis zu 2 Gew. % des Emulgators enthält, zugesetzt werden. In Frage kommende Emulgatoren sind weiter vorne genannt.

Der Saatdispersion wird das Monomerengemisch zugegeben in Mengen von 90 bis 5 Gew. %, vorzugsweise 40 bis 10 Gew. %, bezogen auf die Gesamtmenge von Saatdispersion und Monomerenmischung.

Die Saatpolymerisation kann an sich nach üblichen Verfahren durchgeführt werden. Bevorzugte Ausführungsform des erfindungsgemäßen Verfahrens ist jedoch das Dosierverfahren, bei dem die Monomerenmischung unter ständigem Rühren der Suspension der Latexkerne bei Polymerisationsbedingungen, d.h. bei einer Temperatur von + 10 bis + 120° C, vorzugsweise + 50 bis + 90° C, zugetropft wird.

Anschließend wird das Polymerisat von überschüssigen Monomeren, Resten von Initiator und Emulgator nach bekannten Verfahren befreit. Vorteilhafterweise unterwirft man das Polymerisat einer Dialyse, zum Beispiel gegen $NaHCO_3$-Puffer (0,01 bis 0,05 Gew. %).

Zur Herstellung der erfindungsgemäßen, biologisch aktiven Dispersionspolymere, nachfolgend auch als Latexkonjugat bezeichnet, wird eine Suspension der oben beschriebenen saatpolymerisierten Latexpartikel auf einen pH-Wert unter 5, vorzugsweise unter 3, eingestellt und mit dem zu bindenden immunologisch aktiven Material, wie zum Beispiel Antikörper oder Antigene, inkubiert. Die labilen Bindungen zwischen einer Aminogruppe des Proteins und dem freigesetzten Aldehyd auf dem erfindungsgemäßen Latexpartikel werden nach bekannten Verfahren reduziert. Bevorzugt wird eine Lösung von Natriumcyanborhydrid in neutralem Puffer hierfür verwendet. Man trennt eventuell ungebundenes immunologisch aktives Material oder andere Verunreinigungen aus dem Reaktionsansatz ab. Dies geschieht zweckmäßigerweise durch Zentrifugieren oder Waschen auf geeigneten Membranen.

Die erfindungsgemäßen, saatpolymerisierten Dispersionspolymere zeichnen sich durch eine besondere Stabilität aus. Sie eignen sich zur Herstellung besonders empfindlicher Reagenzien, während bekannte Dispersionen, insbesondere solche mit hoher Nachweisempfindlichkeit, dazu neigen, schon nach relativ

kurzer Zeit unspezifisch zu agglutinieren.

Dies führt bei nephelometrischen oder turbidimetrischen Messungen zu einem Anstieg des Streulicht- bzw. Extinktionssignals. Der Reagenzblindwert eines nach dem Stand der Technik hergestellten Reagenzes steigt demzufolge innerhalb weniger Stunden derart an, daß eine turbidimetrische Messung mit der fixed-time-Methode nicht mehr möglich ist. Die Veränderung einer Referenzkurve, aufgenommen mit einem Reagenz nach dem Stand der Technik bei unterschiedlichen CRP-Konzentrationen, zeigt Figur 1, wobei Kurve a) mit einem Reagenz unmittelbar nach dessen Herstellung und Kurve b) nach dreistündiger Lagerung des Reagenzes erhalten wurde. In Figur 3 ist die entsprechende Referenzkurve mit dem erfindungsgemäßen Reagenz wiedergegeben. Im Gegensatz zum Reagenz, hergestellt nach dem Stand der Technik, weist das erfindungsgemäße Reagenz einen über einen längeren Zeitraum hinweg unverändert niedrigen Reagenzblindwert auf.

Die ausgeprägte Stabilität des erfindungsgemäßen Latex-Reagenzes zeigt sich auch dadurch, daß nach mehrmonatiger (bis mindestens dreimonatiger) Lagerung des als wässrige Dispersion vorliegenden Reagenzes bei Raumtemperatur man nahezu unveränderte Reagenzblindwerte und nahezu identische Referenzkurven erhält.

Ein weiterer Vorteil des erfindungsgemäßen Reagenzes gegenüber dem nach dem Stand der Technik hergestellten zeigt sich auch darin, daß bei Messungen mit verschiedenen Patientenseren der gemessene Wert weitgehend un beeinflußt von der Serummatrix ist. Das bedeutet, daß man für die Messungen wesentlich geringere Serumverdünnungen einsetzen kann als das bei der Bestimmung von z.B. CRP nach dem Stand der Technik erfolgt. Das bedeutet auch, daß damit wesentlich geringere Konzentrationen bestimmter Proteine im Serum erfaßt werden können. In Figur 4 ist die Referenzkurve für Myoglobin im Meßbereich von 100-2.500 µg/l wiedergegeben, die diesen Vorteil des erfindungsgemäßen Reagenzes veranschaulicht.

Für unterschiedliche Patientenproben, die mit bestimmten Mengen von aufgereinigtem Myoglobin aufgestockt worden sind, erhält man mit den Reagenzien nach dem Stand der Technik unterschiedliche Werte (Tabelle 1). Daß auch in dieser Hinsicht mit dem erfindungsgemäßen Latex-Reagenz eine deutliche Verbesserung erzielt wird, zeigt der Vergleich mit Tabelle 2. Die geringe Anfälligkeit gegen Störeffekte zeigt sich auch in dem niedrigen Wert des Variationskoeffizienten beim erfindun gsgemäßen Latex-Reagenz.

Weitere erfindungsgemäße Reagenzien mit entsprechenden vorteilhaften Eigenschaften erhält man, wenn die erfindungsgemäßen Dispersionspolymere mit Antikörpern beispielsweise gegen Lipase, alpha-Fetoprotein (AFP), Ferritin, Thyroxine bindendes Globulin (TBG), Immunglobulin E, $alpha_1$-Microglobulin, $beta_2$-Microglobulin und Schwangerschaft spezifisches $beta_1$-Glycoprotein, Human chorionic Gonadotropin (beta-HCG), beladen werden. Auch monoklonale Antikörper lassen sich zur Herstellung der erfindungsgemäßen Reagenzien vorteilhaft einsetzen.

Gleichermaßen können Dispersionspolymere mit Antigenen, z.B. bakteriellen oder viralen Proteinen wie z.B. Streptolysin O, Streptococcus B Antigen, H. Influenza Antigen, Pneumococcus Antigen, Lues Antigen, Toxoplasmen Antigenen, HBsAg, Rubella Antigen, Herpes Antigen und Tetanus Antigen beladen werden und die entsprechenden Antikörper durch diese antigenbeladenen, erfindungsgemäßen Reagenzien nachgewiesen werden.

Schließlich können in gleicher Weise Dispersionspolymere gemäß der Erfindung mit Haptenen, z.B. derivatisierten Haptenen, z.B. Hormonen oder Arzneimitteln, wie Thyroxin ($T_4$), Trijodothyronin ($T_3$), Cortisol, Progesteron, Testosteron, Gentamycin und Digoxin beladen werden, wodurch man biologisch aktive Dispersionspolymere, also die erfindungsgemäßen Reagenzien, erhält, mit denen man durch Inhibitionsteste, also unter gleichzeitiger Verwendung geeigneter Antikörper, die Konzentration der genannten Haptene messen kann. Diese biologisch aktiven Dispersionspolymere können zum diagnostischen Nachweis von Antigenen, Antikörpern und Haptenen verwendet werden.

Die erfindungsgemäßen Latexpräparationen sind einfach herzustellen und schonend mit empfindlichen immunologisch aktiven Materialien zu einem diagnostischen Reagenz zu verknüpfen. Die erfindungsgemäßen Dispersionspolymere und ihre daraus hergestellten biologisch aktiven Latices, die Latexkonjugate und -reagenzien, sind stabil im Vergleich zu jenen des Standes der Technik. Sie sind unempfindlich gegen Störung durch Matrixeffekte, mit ihnen durchgeführte nephelometrische oder turbidimetrische Messungen führen im Vergleich zum Stand der Technik zu wesentlich weniger falschpositiven oder falschnegativen Ergebnissen. Bei Aufstockung myoglobinfreier Seren mit aufgereinigtem Myoglobin findet man mit dem erfindungsgemäßen Reagenz Werte, die wenig schwanken und nahe dem theoretischen Wert liegen.

Die biologisch aktiven Dispersionspolymere können in allen diagnostischen Verfahren eingesetzt werden, die Teilchengrößenveränderungen messen, beispielsweise in qualitativen und semiquantitativen Bestimmungen von Substanzen mit Hilfe von visuellen Latexagglutinationstesten sowie zu nephelometrischen oder turbidimetrischen Bestimmungen, z.B. von Spurenproteinen im direkten oder kompetitiven Agglutinationstest oder im Latex-Hapten-Inhibitionstest, und für Partikelzählverfahren.

Beispiele

1. Polymerisation von N-(3-Sulfopropyl)-N-methacrylamidopropyl-N,N-dimethyl-ammoniumbetain auf Polystyrolkerne als Saat (Saatpolymere)

In ein zylindrisches Glasgefäß, ausgestattet mit Gasein- und Gasauslaßrohr sowie einem Magnetrührstab, wurden 11,1 ml Polystyrol-Latexsuspension mit einem Feststoffgehalt von 36 % sowie 67,9 ml destilliertes Wasser und 50 mg Natriumdodecylsulfat gegeben und durch Rühren gelöst.

Durch mehrfaches Evakuieren und Füllen mit Stickstoff wurde das Polymerisationsgefäß unter Inertgasatmosphäre gebracht. Die Latexsuspensionsmischung wurde unter ständigem Rühren auf + 70° C in einem Wasserbad erhitzt. Man gab 1 ml einer Kaliumperoxydisulfatlösung (16 mg/ml in destilliertem Wasser) hinzu.

Es wurde eine Monomerenmischung hergestellt aus 0,4 ml Styrol, 0,15 ml Methacrylamidoacetaldehyd-di-n-pentylacetal, 0,01 ml Methacrylsäure und 0,3 ml N-(3-Sulfopropyl)-N-methacrylamidopropyl-N,N-dimethylammoniumbetain sowie zur besseren Löslichkeit dieser Monomeren 1 ml Dimethylformamid.

Unter starkem Rühren der Polystyrol-Latexsuspension wurde zu dieser die Mischung der Monomeren während 60 Minuten langsam hinzugetropft. Die Temperatur des Polymerisationsansatz es wurde auf + 70° C gehalten. Nach dem Zutropfen der Monomeren wurde weitere vier Stunden bei der genannten Temperatur gerührt. Danach wurde die Dispersion auf Raumtemperatur abgekühlt und mittels Faltenfilter filtriert. Man erhielt 74 ml einer Latexsuspension. Diese wurde anschließend 20 Stunden gegen eine Natriumhydrogencarbonat-Pufferlösung (0,25 g/l pH 8,0-8,2) dialysiert. Man erhielt 88 ml einer Latexsuspension mit einem Feststoffgehalt von 4,5 %.

2. Polymerisation von N-(3-Sulfopropyl)-N-methacryloxethyl-N,N-dimethyl-ammoniumbetain auf Polystyrolkerne

Die Polymerisation erfolgte ähnlich wie im Beispiel 1 beschrieben. Man stellte eine Mischung aus 11,1 ml Polystyrol-Latex mit einem Feststoffgehalt von 36 % und 67,9 ml destilliertem Wasser sowie 50 mg Natriumdodecylsulfat her. Diese wurde in das Polymerisationsgefäß gegeben und wie in Beispiel 1 unter Stickstoffatmosphäre gebracht. Weiterhin wurden 1 ml einer Kaliumperoxydisulfat-Lösung (16 mg/ml in destilliertem Wasser) hinzugegeben und der Ansatz auf + 70° C erhitzt. Man stellte eine wässrige Emulsion von 0,4 ml Styrol, 0,2 ml Methacrylamidoacetaldehyd-di-n-pentylacetal und 0,2 mg N-(3-Sulfopropyl)-N-methacryloxyethyl-N,N-dimethylammoniumbetain mit 0,4 ml einer 0,1 %igen Natriumdodecylsulfat-Lösung her.

Diese Emulsion der Monomeren wurde zur kräftig gerührten Polystyrol-Latexdispersion bei + 70° C während 60 Minuten langsam zugetropft. Anschließend wurde weitere vier Stunden bei gleicher Temperatur gerührt. Nach Abkühlung auf Raumtemperatur und Filtration durch einen Faltenfilter erhielt man 76 ml des Polymerisates. Man dialysierte anschließend 22 Stunden gegen Natriumhydrogencarbonat-Puffer (0,25 g/l, pH 8,0-8,2). Man erhielt 81 ml einer Latexdispersion mit einem Feststoffgehalt von 5,1 %.

3a) Bindung von Anti-CRP-Antikörpern an ein Latexpolymerisat

An ein Polymerisat unter Verwendung von N-(3-Sulfopropyl)-N-methacrylamidopropyl-N,N-dimethylammoniumbetain hergestellt nach Beispiel 1 oder unter Verwendung von N-(3-Sulfopropyl)-N-methacryloxethyl-N,N-dimethylammoniumbetain hergestellt nach Beispiel 2, wurden Anti-CRP-Antikörper gebunden.

Das jeweils eingesetzte Polymerisat wurde mit destilliertem Wasser auf einen Feststoffgehalt von 4,5 Gew. % verdünnt. Ein Antiserum, gewonnen durch Immunisierung von Kaninchen mit aufgereinigtem CRP, wurde nach bekannten Verfahren mittels Ionenaustausch-Chromatographie gereinigt. Es wurde anschließend ankonzentriert, bis ein Proteingehalt von 70 mg/ml erreicht war.

2 ml des obengenannten Latexpolymerisats wurden mit 0,057 ml der Anti-CRP-Antikörper-Lösung gemischt. Hierzu gab man 0,06 ml 1 N HCl, so daß ein pH-Wert von ca. 2 erreicht wurde. Nach einer Inkubationszeit von 30 Minuten bei Raumtemperatur gab man 0,5 ml eines 1 M Phosphatpuffers pH 6,5 und 0,5 ml wässrige Natriumcyanborhydrid-Lösung (25 mg/ml) hinzu und mischte gut durch. Anschließend erfolgte eine Inkubation von einer Stunde bei Raumtemperatur. Nach dieser Zeit wurde der Ansatz 2 Stunden mit ca. 50.000 g zentrifugiert (Beckman-Zentrifuge, 20.000 Upm). Der Überstand wurde verworfen. Der Rückstand wurde in 2 ml eines Glycin-NaCl-Puffers (0,1 M Gly, 0,17 M NaCl, 0,5 % Eikosaoxyethylensorbitanlaurylsäureester (® Tween 20), pH 8,2) resuspendiert. Anschließend erfolgte eine Ultraschall-Behandlung (Bronson Sonifier B 15) für 3 Sekunden

3b) Messung von CRP-Konzentrationen in Serumproben

Das nach Beispiel 3a) hergestellte Reagenz zur Bestimmung von CRP wurde mit einem Glycin-NaCl-Puffer (0,1 M Glycin, 0,17 M NaCl, 0,5 Gew. % Eikosaoxyethylensorbitanlaurinsäureester (® Tween 20), pH 8,2), der 4 Gew. % PEG 6000 enthielt, auf einen Feststoffgehalt von 0,0225 % verdünnt. Das so erhaltene Latex-Reagenz wurde gut vermischt und war dann gebrauchsfertig.

Als Standard diente ein LN-CRP-Standard (Fa. Behringwerke AG). Dieses Standard-Serum enthält laut Packungsbeilage 8,6 mg/dl CRP. Dieser Standard wurde mit 0,9 %iger NaCl-Lösung 1:80 verdünnt und dann stufenweise mit 0,9 %iger NaCl-Lösung auf jeweils das doppelte Volumen weiterverdünnt. Man erhielt so eine Standardreihe mit abnehmenden CRP-Konzentrationen.

Zur Messung wurden Patientenseren 1:100 in 0,9 %iger NaCl-Lösung verdünnt und 200 µl Patientenserum-Verdünnung bzw. Standardserum-Verdünnung mit 500 µl des gebrauchsfertigen Latex-Reagenzes in handelsüblichen Küvetten (Fa. Sarstedt, Nr. 67742) gemischt. Die Messung erfolgte nach dem turbidimetrischen Meßprinzip mit der fixed-time-Methode an einem Eppendorf-Photometer 1101 M bei 334 nm. Für jede Küvette wurden die Extinktionswerte nach $t_1 = 15$ sec. Und $t_2 = 300$ sec. Ermittelt. Die Referenzkurve für die Messung der Standardseren wurde auf doppelt-logarithmischem Papier gezeichnet und daran wurden die Meßwerte für die Patientenseren ausgewertet.

Eine typische Referenzkurve ist in Figur 3 dargestellt.

Die Figur 1 zeigt die Referenzkurve für CRP-haltige Seren mit einem Reagenz nach dem Stand der Technik, aufgenommen direkt nach der Herstellung (Kurve a) und nach dreistündiger Lagerung (Kurve b).

In Figur 2 ist die Abhängigkeit der ΔOD-Werte (Differenz der optischen Dichte) von der Meßzeit nach Herstellung des Reagenzes für ein Serum (1:100 verdünnt, Gehalt 24 mg/l CRP) mit einem Reagenz nach dem Stand der Technik.

4a) Bindung von Anti-Myoglobin-Antikörpern an ein Latexpolymerisat

An ein Polymerisat unter Verwendung von N-(3-Sulfopropyl)-N-methacrylamidopropyl-N,N-dimethylammoniumbetain hergestellt nach Beispiel 1 oder unter Verwendung von N-(3-Sulfopropyl)-N-methacryloxethyl-N,N-dimethylammoniumbetain hergestellt nach Beispiel 2 wurden Anti-Myoglobin-Antikörper gebunden.

Das jeweils eingesetzte Polymerisat wurde mit destilliertem Wasser auf einen Feststoffgehalt von 4,5 Gew. % verdünnt. Ein Antiserum, gewonnen durch Immunisierung von Kaninchen mit aufgereinigtem Myoglobin wurde nach bekannten Verfahren mittels Affinitätschromatographie gereinigt. Es wurde anschließend ankonzentriert, bis ein Proteingehalt von 10 mg/ml erreicht war.

2 ml des obengenannten Latexpolymerisats wurden mit 0,35 ml der Anti-Myoglobin-Antikörper-Lösung gemischt. Hierzu gab man 0,06 ml 1 N HCl, so daß ein pH-Wert von ca. 2 erreicht wurde. Nach einer Inkubationszeit von 30 Minuten bei Raumtemperatur gab man 0,5 ml eines 1 M Phosphatpuffers pH 6,5 und 0,5 ml wässrige Natriumcyanborhydrid-Lösung (25 mg/ml) hinzu und mischte gut durch. Anschließend erfolgte eine Inkubation von einer Stunde bei Raumtemperatur. Nach dieser Zeit wurde der Ansatz 2 Stunden mit ca. 50.000 g zentrifugiert (Beckman-Zentrifuge, 20.000 Upm). Der Überstand wurde verworfen.

Der Rückstand wurde in 2 ml eines Glycin-NaCl-Puffers (0,1 M Gly, 0,17 M NaCl, 0,5 % Eikosaoxyethylensorbitanlaurinsäureester (®Tween 20), pH 8,2) resuspendiert. Anschließend erfolgte eine Ultraschall-Behandlung (Bronson-Sonifier B 15) für 3 Sekunden.

4b) Messung von Myoglobin-Konzentration in Serumproben

Das nach Beispiel 4a) hergestellte Reagenz zur Bestimmung von Myoglobin wurde mit einem 0,05 M Phosphatpuffer pH 7,2, der 0,17 M NaCl, 0,1 Gew. % Octylphenoltetrakosaethylenglykolether (®Triton X-405) und 4 Gew. % PEG 6000 enthielt, auf einen Feststoffgehalt von 0,0225 % verdünnt. Das so erhaltene Latex-Reagenz wurde gut vermischt und war dann gebrauchsfertig.

Zur Messung wurden 9,445 ml Myoglobin-freies Humanserum mit 0,555 ml einer 4,5 %igen Lösung von Myoglobin (spektrophotometrisch bestimmt) in 0,9 %iger NaCl-Lösung versetzt und gut vermischt. Dieses als Myoglobin-Standard dienende Serum wurde mit myoglobinfreiem Humanserum stufenweise auf jeweils das doppelte Volumen weiterverdünnt. Man erhielt so eine Standardreihe mit abnehmenden

Myoglobin-Konzentrationen. Die Standardseren wurden 1:5 in einer 0,9 %igen NaCl-Lösung verdünnt und 200 µl der Serumver dünnung mit 500 µl des gebrauchsfertigen Latex-Reagenzes in handelsüblichen Halbmikroküvetten gemischt. Die Messung erfolgte nach dem turbidimetrischen Meßprinzip mit der fixed-time-Methode an einem Eppendorf-Photometer 1101 M bei 334 nm. Für jede Küvette wurden die Extinktionswerte nach $t_1 = 15$ sec. und $t_2 = 300$ sec. ermittelt. Die Referenzkurve für die Messung der Standardseren wurde auf doppelt-logarithmischem Papier gezeichnet. Eine typische Referenzkurve ist in Figur 4 dargestellt.

Tabelle 1

Meßwerte im turbidimetrischen Test, Stand der Technik

| Serum Nr. | Gehalt Myoglobin in µg/l |
|---|---|
| 1 | 213 |
| 2 | 246 |
| 3 | 213 |
| 4 | 213 |
| 5 | 178 |
| 6 | 141 |
| 7 | 213 |
| 8 | 141 |
| 9 | 213 |
| 10 | 213 |
| 11 | 141 |
| 12 | 178 |
| 13 | 178 |
| 14 | 141 |
| 15 | 178 |
| 16 | 141 |

Mittelwert = 183 µg/l = 91 % Wiederfindung
Sollwert = 200 µg/l
Variationskoeffizient = 19 %

Meßwerte im turbidimetrischen Test nach Beispiel 4b) für Serumproben, die mit jeweils 200 µg/l Myoglobin aufgestockt worden sind. Die Messungen erfolgten mit einem Reagenz, hergestellt nach dem Stand der Technik.

Tabelle 2

Meßwerte im turbidimetrischen Test, erfindungsgemäßes Reagenz

| Serum Nr. | Gehalt Myoglobin in µg/l |
|---|---|
| 1 | 201 |
| 2 | 216 |
| 3 | 231 |
| 4 | 201 |
| 5 | 146 |
| 6 | 216 |
| 7 | 201 |
| 8 | 216 |
| 9 | 231 |
| 10 | 185 |
| 11 | 246 |
| 12 | 231 |
| 13 | 216 |
| 14 | 231 |
| 15 | 246 |
| 16 | 201 |

Mittelwert = 220 µg/l = 110 % Wiederfindung
Sollwert = 200 µg/l
Variationskoeffizient = 8,6 %

Meßwerte im turbidimetrischen Test nach Beispiel 4b) für Serumproben, die mit jeweils 200 µg/l Myoglobin aufgestockt worden sind. Die Messungen erfolgten mit einem erfindungsgemäßen Reagenz.

**Patentansprüche**
**Patentansprüche für folgende Vertragsstaaten : BE, CH, DE, FR, GB, IT, LI, LU, NL, SE**

1. Dispersionspolymere, bestehend aus Latexteilcnen, in einem wässrigen Medium auf deren Oberfläche sich ein Copolymerisat befindet, das Monomeren mit endständigen Acetalen der Formel I

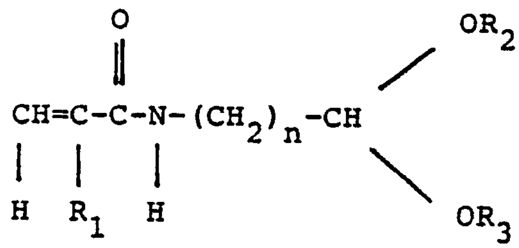

Formel I

worin
$n = 1 - 6$;
$R_1 = H, CH_3$ und
$R_2$ und $R_3$ gleich oder unterschiedlich sind mit
$R_2, R_3 = -(CH_2)_m-CH_3$ $m = 0-7$ oder

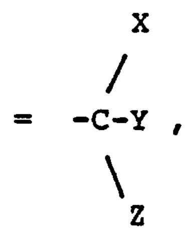

wobei X, Y, Z = $(CH_2)_p-CH_3$ und $p = 1-3$ sind,
mit X, Y, Z gleich oder unterschiedlich

und Monomeren der Formel II

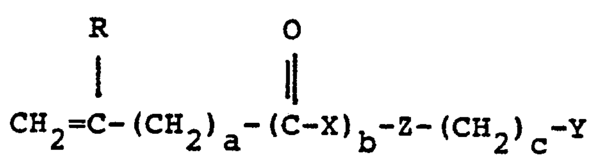

Formel II

wobei
$R = H, CH_3$
$X = -NH, -O$
$Y = SO_3^-, COO^-$

$$Z = -(CH_2)_k-{}^{+}N[(CH_2)_m-H]_2-$$

mit $k = 1-12$

$m = 1-6$

oder

Z = 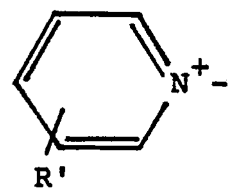 mit R' = H, 1-3 C-alkyl

a = 0- 6
b = 0- 1
c = 1-12
enthält.

2. Dispersionspolymere nach Anspruch 1, erhältlich durch Polymerisation einer Monomerenmischung aus einer Verbindung der Formel I und einer Verbindung der Formel II in Gegenwart einer Saatdispersion in wässrigem Medium.

3. Dispersionspolymere nach Anspruch 1, wobei als Monomer der Formel II N-(omega-Sulfoalkyl)-N-acrylamidoalkylN,N-dialkyl-ammoniumbetain, N-(omega-Sulfoalkyl)-N-methacrylamidoalkyl-N,N-dialkylammoniumbetain oder die entsprechenden N-(omega-Carboxyalkyl)-Verbindungen eingesetzt sind.

4. Dispersionspolymere nach Anspruch 1, wobei als Monomer der Formel II eingesetzt ist, wenigstens eine der Verbindungen N-(3-Sulfopropyl)-N-methacryloxyethyl-N, N-dimethylammoniumbetain, N-(3-Sulfopropyl)-N-methacrylamidopropyl-N,N-dimethylammoniumbetain oder die entsprechenden Acrylverbindungen.

5. Dispersionspolymere nach Anspruch 1, wobei als Monomer der Formel II 1(3-Sulfopropyl)-2-vinyl-pyridiniumbetain eingesetzt ist.

6. Dispersionspolymere nach Anspruch 1, wobei als Monomere der Formel I Acryl- oder Methacrylamidoalkylaldehyd-di-alkylacetal mit Alkyl = $C_1$ bis $C_8$ eingesetzt sind.

7. Dispersionspolymere nach Anspruch 1, worin als Monomer der Formel I Methacrylamidoacetaldehyd-di-n-pentylacetal verwendet wird.

8. Verfahren zur Herstellung von Dispersionspolymeren nach Anspruch 1, dadurch gekennzeichnet, daß zu einer Saatdispersion, bestehend aus Latex teilchen in einem wässrigen Medium mit einem Partikeldurchmesser von 0,02 bis 2 $\mu m$ Emulgator und Radikalstarter zugesetzt werden und sodann unter Rühren bei einer Temperatur von + 10° C bis + 120° C eine Monomerenmischung zugetropft wird, die eine Verbindung der Formel II und eine Verbindung der Formel I enthält.

9. Verfahren nach Anspruch 8, dadurch gekennzeichnet, daß die Monomerenmischung als Verbindung der Formel II enthält N-(omega-Sulfoalkyl)-N-acrylamidoalkyl-N,N-dialkylammoniumbetain, N-(omega-Sulfoalkyl)-N-methacrylamidoalkyl-N,N-dialkylammoniumbetain oder die entsprechenden N-(omega-Carboxyalkyl)-Verbindungen und als Verbindung der Formel I Acryl-oder Methacrylamidoalkyl-aldehyd-di-alkylacetal mit Alkyl = $C_1$ bis $C_8$ eingesetzt wird.

10. Verfahren nach Anspruch 8, dadurch gekennzeichnet, daß die Monomerenmischung als Verbindung der Formel II enthält N-(3-Sulfopropyl)-N-methacryloxyethyl-N, N-dimethylammoniumbetain, N-(3-Sulfopropyl)-N-methacrylamidopropyl-N,N-dimethylammoniumbetain oder die entsprechenden Acrylverbindungen und als Verbindung der Formel I eingesetzt wird Acryl- oder Methacrylamidoalkyl-aldehyd-di-n-pentylacetal.

11. Verfahren nach Anspruch 8, dadurch gekennzeichnet, daß die Monomerenmischung als Verbindung II 1(3-Sulfopropyl)-2-vinyl-pyridiniumbetain in enthält und als Verbindung der Formel I eingesetzt. wird Acryl- oder Methacrylamidoalkyl-aldehyd-di-n-pentylacetal.

12. Verfahren gemäß Anspruch 8, dadurch gekennzeichnet, daß die Monomerenmischung zusätzlich bis zu 50 Gew. %, bezogen auf die Gesamtmischung, Styrol, Vinylnaphthalin oder Vinyltoluol enthält.

13. Verfahren nach Anspruch 8, dadurch gekennzeichnet, daß das Monomerengemisch zusätzlich bis zu 30 Gew. %, bezogen auf die Gesamtmischung, Methacrylsäure, Acrylsäure oder Crotonsäure enthält.

14. Verfahren nach Anspruch 8, dadurch gekennzeichnet, daß die Monomerenmischung zusätzlich zu Monomeren der Formel I und II sowie Verbindungen aus der Gruppe Styrol, Vinylnaphthalin oder Vinyltoluol und einer der Verbindungen Methacrylsäure, Acrylsäure oder Crotonsäure zusätzlich noch bis zu 90 Gew. %, bezogen auf die Gesamtmischung, Dimethylformamid enthält.

15. Verfahren nach Anspruch 8, dadurch gekennzeichnet, daß die Monomerenmischung zusätzlich zu Monomeren der Formel I und II sowie Verbindungen aus der Gruppe Styrol, Vinylnaphthalin oder Vinyltoluol und einer der Verbindungen Methacrylsäure, Acrylsäure oder Crotonsäure zusätzlich noch bis zu 90 Gew. %, bezogen auf die Gesamtmischung einer bis zu 2 %igen wässrigen Emulgatorlösung enthält.

16. Biologisch aktive Dispersionspolymere, bestehend aus Dispersionspolymeren gemäß Anspruch 1 und daran gebundene Antikörper, Antigene oder Haptene.

17. Verwendung eines biologisch aktiven Dispersionspolymeren nach Anspruch 16 zum diagnostischen Nachweis von Antigenen, Antikörpern oder Haptenen.

18. Verwendung eines biologisch aktiven Dispersionspolymeren nach Anspruch 16 in nephelometrischem und turbidimetrischem Verfahren sowie für Partikelzählverfahren.

19. Diagnostisches Mittel enthaltend Dispersionspolymere gemäß Anspruch 1 und daran gebundene Antikörper, Antigene oder Haptene.

**Patentansprüche für folgende Vertragsstaaten : ES, AT**

1. Verfahren zur Herstellung von Dispersionspolymeren, dadurch gekennzeichnet, daß zu einer Saatdispersion, bestehend aus Latex teilchen in einem wässrigen Medium mit einem Partikeldurchmesser von 0,02 bis 2 µm Emulgator und Radikalstarter zugesetzt werden und sodann unter Rühren bei einer Temperatur von + 10° C bis + 120° C eine Monomerenmischung zugetropft wird, die eine Verbindung der Formel I

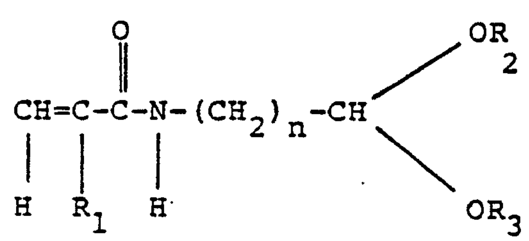

Formel I

worin
n = 1 - 6;
$R_1$ = H, $CH_3$ und
$R_2$ und $R_3$ gleich oder unterschiedlich sind mit

$R_2$, $R_3$ = $-(CH_2)_m-CH_3$ m = 0-7 oder

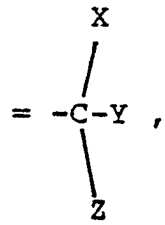

wobei X, Y, Z = $(CH_2)_p-CH_3$ und p = 1-3 sind,
mit X, Y, Z gleich oder unterschiedlich

und eine Verbindung der Formel II

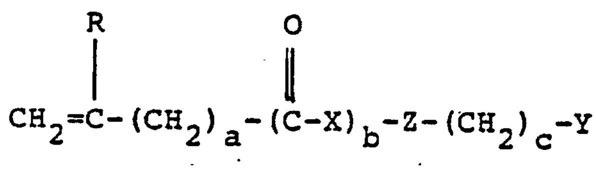

Formel II

wobei
R = H, $CH_3$
X = -NH, -O
Y = $SO_3^-$, $COO^-$

$$Z = -(CH_2)_k-{}^{+}N\left[(CH_2)_m-H\right]_2-$$

mit k = 1-12
m = 1- 6

oder

Z = 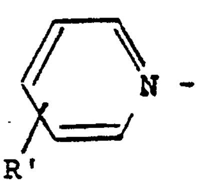 mit R' = H, 1-3 C-alkyl

a = 0- 6
b = 0- 1
c = 1-12.
enthält.

2. Verfahren nach Anspruch 1, dadurch gekennzeichnet, daß die Monomerenmischung als Verbindung der Formel II enthält N-(omega-Sulfoalkyl)-N-acrylamicoalkyl-N,N-dialkylanmoniumbetain, N-(omega-Sulfoalkyl)-N-methacrylamidoalkyl-N,N-dialkylammoniumbetain oder die entsprechenden N-(omega-Carboxyalkyl)-Verbindungen und als Verbindung der Formel I Acryl- oder Methacrylamidoalkyl-aldehyd-dialkylacetal mit Alkyl = $C_1$ bis $C_1$ eingesetzt wird.

3. Verfahren nach Anspruch 1, dadurch gekennzeichnet, daß die Monomerenmischung als Verbindung der Formel II enthält N-(3-Sulfopropyl)-N-methacryloxyethyl-N, N-dimethylammoniumbetain, N-(3-Sulfopropyl)-N-methacrylamidopropyl-N,N-dimethylammoniumbetain oder die entsprechenden Acrylverbindungen und als Verbindung der Formel I eingesetzt wird Acryl- oder Methacrylamidoalkyl-aldehyd-di-n-pentylacetal.

4. Verfahren nach Anspruch 1, dadurch gekennzeichnet, daß die Monomerenmischung als Verbindung II 1(3-Sulfopropyl)-2-vinyl-pyridiniumbetain enthält und als Verbindung der Formel I eingesetzt wird Acryl- oder Methacrylamidoalkyl-aldehyd-di-n-pentylacetal.

5. Verfahren gemäß Anspruch 1, dadurch gekennzeichnet, daß die Monomerenmischung zusätzlich bis zu 50 Gew. %, bezogen auf die Gesamtmischung, Styrol, Vinylnaphthalin oder Vinyltoluol enthält.

6. Verfahren nach Anspruch 1, dadurch gekennzeichnet, daß das Monomerengemisch zusätzlich bis zu 30 Gew. %, bezogen auf die Gesamtmischung, Methacrylsäure, Acrylsäure oder Crotonsäure enthält.

7. Verfahren nach Anspruch 1, dadurch gekennzeichnet, daß die Monomerenmischung zusätzlich zu Monomeren der Formel I und II sowie Verbindungen aus der Gruppe Styrol, Vinylnaphthalin oder Vinyltoluol und einer der Verbindungen Methacrylsäure, Acrylsäure oder Crotonsäure zusätzlich noch bis zu 90 Gew. %, bezogen auf die Gesamtmischung, Dimethylformamid enthält.

8. Verfahren nach Anspruch 1, dadurch gekennzeichnet, daß die Monomerenmischung zusätzlich zu Monomeren der Formel I und II sowie Verbindungen aus der Gruppe Styrol, Vinylnaphthalin oder Vinyltoluol und einer der Verbindungen Methacrylsäure, Acrylsäure oder Crotonsäure zusätzlich noch bis zu 90 Gew. %, bezogen auf die Gesamtmischung einer bis zu 2 %igen wässrigen Emulgatorlösung enthält.

9. Verfahren zur Herstellung biologisch aktiver Dispersionspolymere, dadurch gekennzeichnet, daß man Dispersionspolymere nach Anspruch 1 herstellt und daran Antikörper, Antigene oder Haptene bindet.

10. Verfahren zum Nachweis von Antigenen, Antikörpern oder Haptenen, dadurch gekennzeichnet, daß man nach Anspruch 9 hergestellte biologisch aktive Dispersionspolymere verwendet.

11. Verfahren nach Anspruch 10, dadurch gekennzeichnet, daß nephelometrische oder turbidimetrische Verfahren oder Partikelzählverfahren zum Nachweis angewendet werden.

**Claims**
**Claims for the following Contracting States : BE, CH, DE, FR, GB, IT, LI, LU, NL, SE**

1. A dispersion polymer composed of latex particles in an aqueous medium, on the surface of which particles is located a copolymer which contains monomers having terminal acetals of the formula I

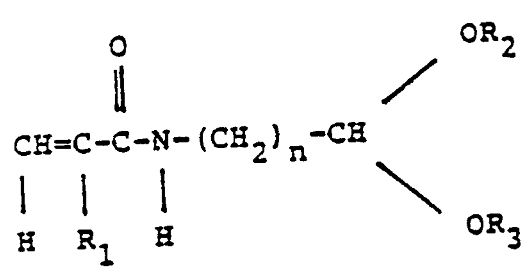

in which
n = 1-6;
$R_1$ = H or $CH_3$, and
$R_2$ and $R_3$ are identical or different with
$R_2$ and $R_3$ = $-(CH_2)_m-CH_3$ m = 0-7 or

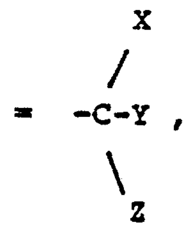

X, Y and Z being $(CH_2)_p-CH_3$ and p being 1-3, with X, Y, Z identical or different,

and monomers of the formula II

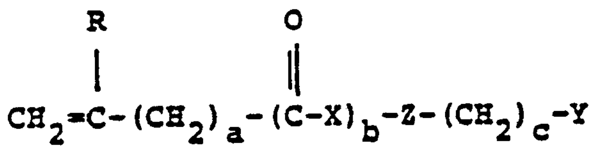

Formula II

with
R = H or $CH_3$
X = -NH or -O,
Y = $SO_3^-$ or $COO^-$

$$Z = -(CH_2)_k-{}^+N[(CH_2)_m-H]_2$$ - with k = 1-12, m = 1- 6

or

Z = 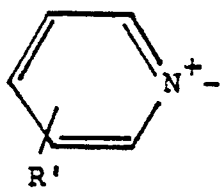 with R' = H or $C_1$-$C_3$-alkyl

with R' = H or $C_1$-$C_3$-alkyl a = 0-6
b = 0-1
c = 1-12

2. A dispersion polymer as claimed in claim 1, obtainable by polymerization of a monomer mixture composed of a compound of the formula I and of a compound of the formula II in the presence of a seed dispersion in aqueous medium.

3. A dispersion polymer as claimed in claim 1, the monomer of the formula II which is used being N-(omega-sulfoalkyl)-N-acrylamidoalkyl-N,N-dialkylammonium betaine, N-(omega-sulfoalkyl)-N-methacrylamidoalkyl-N,N-dialkylammonium betaine or the corresponding N-(omega-carboxyalkyl) compounds.

4. A dispersion polymer as claimed in claim 1, the monomer of the formula II which is used being at least one of the compounds N-(3-sulfopropyl)-N-methacryloxyethyl-N,N-dimethylammonium betaine, N-(3-sulfopropyl)-N-methacrylamidopropyl-N,N-dimethylammonium betaine or the corresponding acrylic compounds.

5. A dispersion polymer as claimed in claim 1, the monomer of the formula II which is used being 1-(3-sulfopropyl)-2-vinylpyridinium betaine.

6. A dispersion polymer as claimed in claim 1, the monomer of the formula I which is used being acryl-or methacrylamidoalkylaldehyde dialkyl acetal where alkyl = $C_1$ to $C_8$.

7. A dispersion polymer as claimed in claim 1, in which the monomer of the formula I which is used is methacrylamidoacetaldehyde di-n-pentyl acetal.

8. A process for the preparation of dispersion polymers as claimed in claim 1, which comprises addition of an emulsifier and radical initiator to a seed dispersion composed of latex particles in an aqueous medium with a particle diameter of 0.02 to 2 µm, and then dropwise addition, while stirring at a temperature of +10° C to +120° C, of a monomer mixture which contains a compound of the formula II and a compound of the formula I.

9. The process as claimed in claim 8, wherein the monomer mixture contains as compound of the formula II N-(omega-sulfoalkyl)-N-acrylamidoalkyl-N,N-dialkylammonium betaine, N-(omega-sulfoalkyl)-N-methacrylamidoalkyl-N,N-dialkylammonium betaine or the corresponding N-(omega-carboxyalkyl) compounds, and the compound of the formula I which is used is acryl- or methacrylamidoalkylaldehyde dialkyl acetal with alkyl = $C_1$ to $C_8$.

10. The process as claimed in claim 8, wherein the monomer mixture contains as compound of the formula II N-(3-sulfopropyl)-N-methacryloxyethyl-N,N-dimethylammonium betaine, N-(3-sulfopropyl)-N-methacrylamidopropyl-N,N-dimethylammonium betaine or the corresponding acrylic compounds, and the compound of the formula I which is used is acryl- or methacrylamidoalkylaldehyde di-n-pentyl acetal.

11. The process as claimed in claim 8, wherein the monomer mixture contains as compound II 1-(3-sulfopropyl)-2-vinylpyridinium betaine, and the compound of the formula I which is used is acryl- or methacrylamidoalkylaldehyde di-n-pentyl acetal.

12. The process as claimed in claim 8, wherein the monomer mixture additionally contains up to 50% by weight, based on the total mixture, of styrene, vinylnaphthalene or vinyltoluene.

13. The process as claimed in claim 8, wherein the monomer mixture additionally contains up to 30% by weight, based on the total mixture, of methacrylic acid, acrylic acid or crotonic acid.

14. The process as claimed in claim 8, wherein the monomer mixture contains, in addition to monomers of the formula I and II, as well as compounds from the group comprising styrene, vinylnaphthalene or vinyltoluene and one of the compounds methacrylic acid, acrylic acid or crotonic acid, also additionally up to 90% by weight, based on the total mixture, of dimethylformamide.

15. The process as claimed in claim 8, wherein the monomer mixture contains, in addition to monomers of the formula I and II, as well as compounds from the group comprising styrene, vinylnaphthalene or vinyltoluene and one of the compounds methacrylic acid, acrylic acid or crotonic acid, also additionally up to 90% by weight, based on the total mixture, of an aqueous emulsifier solution which is up to 2% strength.

16. A biologically active dispersion polymer composed of a dispersion polymer as claimed in claim 1 and, bound thereto, antibodies, antigens or haptens.

17. The use of a biologically active dispersion polymer as claimed in claim 16 for the diagnostic detection of antigens, antibodies or haptens.

18. The use of a biologically active dispersion polymer as claimed in claim 16 in nephelometric and turbidimetric procedures and for particle counting procedures.

19. A diagnostic aid containing a dispersion polymer as claimed in claim 1 and, bound thereto, antibodies, antigens or haptens.

**Claims for the following Contracting States : ES, AT**

1. A process for the preparation of a dispersion polymer, which comprises addition of an emulsifier and radical initiator to a seed dispersion composed of latex particles in an aqueous medium with a particle diameter of 0.02 to 2 $\mu m$, and then dropwise addition, while stirring at a temperature of + 10° C to + 120° C, of a monomer mixture which contains a compound of the formula I

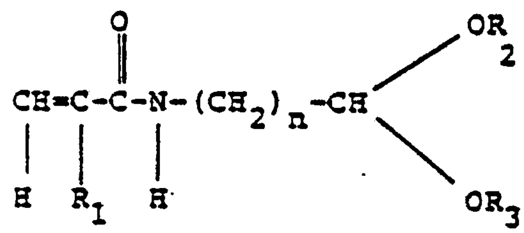

Formula I

in which
n = 1-6;
$R_1$ = H or $CH_3$, and
$R_2$ and $R_3$ are identical or different with
$R_2$ and $R_3$ = $-(CH_2)_m-CH_3$ m = 0-7 or

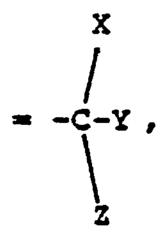

X, Y and Z being $(CH_2)_p-CH_3$ and p being 1-3,
with X, Y, Z identical or different,

and a compound of the formula II

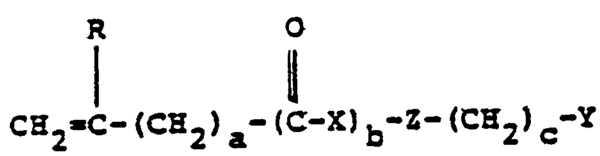

Formula II

with
R = H or $CH_3$,
X = -NH or O,

$$Z = -(CH_2)_k-\overset{+}{N}\left[(CH_2)_m-H\right]_2- \quad \text{with } k = 1\text{-}12,\ m = 1\text{-}6,$$

or

Z = 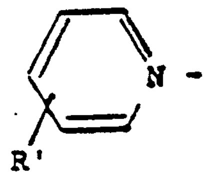 with R' = H or $C_1$-$C_3$-alkyl

Y = $SO_3^-$ or $COO^-$ with R' = H or $C_{1-3}$-alkyl a = 0-6
b = 0-1
c = 1-12.

2. The process as claimed in claim 1, wherein the monomer mixture contains as compound of the formula II N-(omega-sulfoalkyl)-N-acrylamidoalkyl-N,N-dialkylammonium betaine, N-(omega-sulfoalkyl)-N-methacrylamidoalkyl-N,N-dialkylammonium betaine or the corresponding N-(omega-carboxyalkyl) compounds, and the compound of the formula I which is used is acryl- or methacrylamidoalkylaldehyde dialkyl acetal with alkyl = $C_1$ to $C_8$.

3. The process as claimed in claim 1, wherein the monomer mixture contains as compound of the formula II N-(3-sulfopropyl)-N-methacryloxyethyl-N,N-dimethylammonium betaine, N-(3-sulfopropyl)-N-methacrylamidopropyl-N,N-dimethylammonium betaine or the corresponding acrylic compounds, and the compound of the formula I which is used is acryl- or methacrylamidoalkylaldehyde di-n-pentyl acetal.

4. The process as claimed in claim 1, wherein the monomer mixture contains as compound II 1-(3-sulfopropyl)-2-vinylpyridinium betaine, and the compound of the formula I which is used is acryl- or methacrylamidoalkylaldehyde di-n-pentyl acetal.

5. The process as claimed in claim 1, wherein the monomer mixture additionally contains up to 50% by weight, based on the total mixture, of styrene, vinylnaphthalene or vinyltoluene.

6. The process as claimed in claim 1, wherein the monomer mixture additionally contains up to 30% by weight, based on the total mixture, of methacrylic acid, acrylic acid or crotonic acid.

7. The process as claimed in claim 1, wherein the monomer mixture contains, in addition to monomers of the formula I and II, as well as compounds from the group comprising styrene, vinylnaphthalene or vinyltoluene and one of the compounds methacrylic acid, acrylic acid or crotonic acid, also additionally up to 90% by weight, based on the total mixture, of dimethylformamide.

8. The process as claimed in claim 1, wherein the monomer mixture contains, in addition to monomers of the formula I and II, as well as compounds from the group comprising styrene, vinylnaphthalene or vinyltoluene and one of the compounds methacrylic acid, acrylic acid or crotonic acid, also additionally up to 90% by weight, based on the total mixture, of an aqueous emulsifier solution which is up to 2% strength.

9. A process for the preparation of a biologically active dispersion polymer, which comprises a dispersion polymer as claimed in claim 1 being prepared and antibodies, antigens or haptens being bound thereto.

10. A method for detecting antigens, antibodies or haptens, wherein a biologically active dispersion polymer prepared as claimed in claim 9 is used.

11. The method as claimed in claim 10, wherein nephelometric or turbidimetric procedures or particle

counting procedures are used for detection.

## Revendications

**Revendications pour les Etats contractants suivants : BE, CH, DE, FR, GB, IT, LI, LU, NL, SE**

1. Polymères en dispersion constitués de particules de latex dans un milieu aqueux, à la surface desquelles se trouve un copolymère qui comporte des monomères à acétals terminaux, de formule I

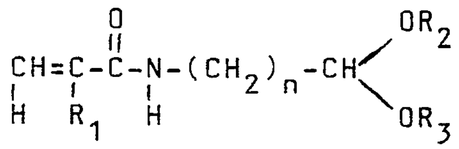

Formule I

dans laquelle
$n = 1\text{-}6$;
$R_1$ = H ou $CH_3$ et
$R_2$ et $R_3$ sont identiques ou différents, avec
$R_2$, $R_3$ = $-(CH_2)_m-CH_3$ m = 0-7, ou

$R_2$, $R_3$ = 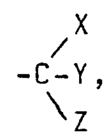

X, Y, Z étant $(CH_2)_p-CH_3$ et
p = 1-3,
X, Y, Z étant identiques ou différents,
et des monomères de formule II

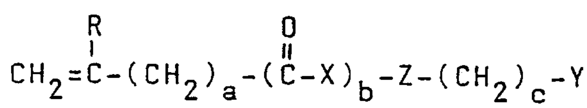

Formule II

dans laquelle
R = H ou $CH_3$
X = -NH ou -O
Y = $SO_3^-$ ou $COO^-$
Z = $-(CH_2)_k-\overset{+}{N}[(CH_2)_m-H]_2-$, avec k = 1-12
m = 1-6
ou

Z = 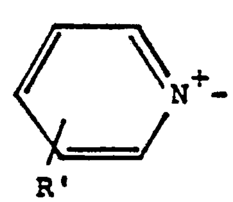

R' représentant H ou un groupe alkyle en $C_1$-$C_3$
a = 0-6
b = 0-1
c = 1-12

2. Polymères en dispersion selon la revendication 1, préparables par polymérisation d'un mélange de

monomères constitué d'un composé de formule I et d'un composé de formule II, en présence d'une dispersion d'ensemencement en milieu aqueux.

**3.** Polymères en dispersion selon la revendication 1, dans lesquels on utilise en tant que monomère de formule II une N-(oméga-sulfoalkyl)-N-acrylamidoalkyl-N,N-dialkylammoniumbétaïne,une N-(oméga-sulfoalkyl)-N-méthacrylamidoalkyl-N,N-dialkylammoniumbétaïne ou les composés N-(oméga-carboxyalkyle) correspondants.

**4.** Polymères en dispersion selon la revendication 1, dans lesquels on utilise en tant que monomère de formule II au moins un des composés N-(3-sulfopropyl)-N-méthacryloxyéthyl-N,N-diméthylammoniumbétaïne, N-(3-sulfopropyl)-N-méthacrylamidopropyl-N,N- diméthylammoniumbétaïne ou les composés acryliques correspondants.

**5.** Polymères en dispersion selon la revendication 1, dans lesquels on utilise en tant que monomère de formule II la 1-(3-sulfopropyl)-2-vinyl-pyridiniumbétaïne.

**6.** Polymères en dispersion selon la revendication 1, dans lesquels on utilise en tant que monomère de formule I un acryl- ou méthacrylamidoalkylaldéhyde-dialkylacétal dont les groupes alkyle sont en $C_1$-$C_8$.

**7.** Polymères en dispersion selon la revendication 1, dans lesquels on utilise en tant que monomère de formule I le méthacrylamidoacétaldéhyde-di-n-pentylacétal.

**8.** Procédé pour la préparation des polymères en dispersion selon la revendication 1, caractérisé en ce que l'on ajoute un émulsifiant et un initiateur de radicaux à une dispersion d'ensemencement constitués de particules de latex dans un milieu aqueux, ayant un diamètre de particules de 0,02 à 2 µm, et on y ajoute ensuite goutte à goutte, sous agitation, à une température de +10 à +120 °C, un mélange de monomères qui contient un composé de formule II et un composé de formule I.

**9.** Procédé selon la revendication 8, caractérisé en ce que le mélange de monomères contient, en tant que composé de formule II, une N-(oméga-sulfoalkyl)-N-acrylamidoalkyl-N,N-dialkylammoniumbétaïne, une N-(oméga-sulfoalkyl)-N-méthacrylamidoalkyl-N,N-dialkylammoniumbétaïne ou les composés N-(oméga-carboxyalkyle) correspondants et on utilise en tant que composé de formule I un acryl- ou méthacrylamidoalkylaldéhyde-dialkylacétaldont les groupes alkyle sont en $C_1$ à $C_8$.

**10.** Procédé selon la revendication 8, caractérisé en ce que le mélange de monomères contient, en tant que composé de formule II, la N-(3-sulfopropyl)-N-méthacryloxyéthyl-N,N-diméthylammoniumbétaïne, la N-(3-sulfopropyl)-N-méthacrylamidopropyl-N,N-diméthylammoniumbétaïne ou les composés acryliques correspondants, et on utilise en tant que composé de formule I un acryl- ou méthacrylamidoalkylaldéhyde-di-n-pentylacétal.

**11.** Procédé selon la revendication 8, caractérisé en ce que le mélange de monomères contient en tant que composé II la 1-(3-sulfopropyl)-2-vinyl-pyridiniumbétaine, et on utilise en tant que composé de formule I un acryl- ou méthacrylamidoalkyl-aldéhyde-di-n-pentylacétal.

**12.** Procédé selon la revendication 8, caractérisé en ce que le mélange de monomères contient en outre jusqu'à 50 % en poids, par rapport au mélange total, de styrène, vinylnaphtalène ou vinyltoluène.

**13.** Procédé selon la revendication 8, caractérisé en ce que le mélange de monomères contient en outre jusqu'à 30 % en poids, par rapport au mélange total, d'acide méthacrylique, acide acrylique ou acide crotonique.

**14.** Procédé selon la revendication 8, caractérisé en ce qu'en plus des monomères de formule I et II ainsi que de composés choisis parmi le styrène, le vinylnaphtalène et le vinyltoluène et l'un des composés acide méthacrylique, acide acrylique ou acide crotonique, le mélange de monomères contient jusqu'à 90 % en poids, par rapport au mélange total, de diméthylformamide.

**15.** Procédé selon la revendication 8, caractérisé en ce qu'en plus des monomères de formule I et II ainsi

que de composés choisis parmi le styrène, le vinylnaphtalène et le vinyltoluène et l'un des composés acide méthacrylique, acide acrylique ou acide crotonique, le mélange de monomères contient jusqu'à 90 % en poids, par rapport au mélange total, d'une solution aqueuse d'émulsifiant à jusqu'à 2 %.

**16.** Polymères en dispersion biologiquement actifs, constitués de polymères en dispersion selon la revendication 1 et d'anticorps, antigènes ou haptènes liés à ceux-ci.

**17.** Utilisation d'un polymère en dispersion biologiquement actif selon la revendication 16, pour la détermination diagnostique d'antigènes, anticorps ou haptènes.

**18.** Utilisation d'un polymère en dispersion biologiquement actif selon la revendication 16, dans le procédé néphélométrique ou turbidimétrique ainsi que pour des procédés de comptage de particules.

**19.** Agent diagnostique contenant des polymères en dispersion selon la revendication 1 et des anticorps, antigènes ou haptènes liés à ceux-ci.

**Revendications pour les Etats contractants suivants : ES, AT**

**1.** Procédé pour la préparation de polymères en dispersion, caractérisé en ce que l'on ajoute un émulsifiant et un initiateur de radicaux à une dispersion d'ensemencement, constituée de particules de latex dans un milieu aqueux, ayant un diamètre de particules de 0,02 à 2 µm, et on y ajoute ensuite goutte à goutte, sous agitation, à une température de +10 à +120° C, un mélange de monomères qui contient un composé de formule I

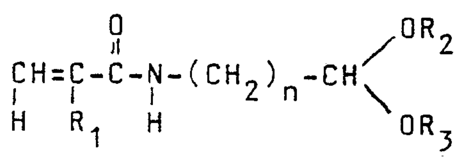

Formule I

dans laquelle
$n = 1-6$;
$R_1 = H$ ou $CH_3$ et
$R_2$ et $R_3$ sont identiques ou différents, avec
$R_2, R_3 = -(CH_2)_m-CH_3$ $m = 0-7$, ou

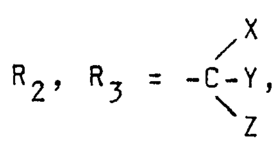

X, Y, Z étant $(CH_2)_{-p}CH_3$ et
$p = 1-3$,
X, Y, Z étant identiques ou différents,
et un composé de formule II

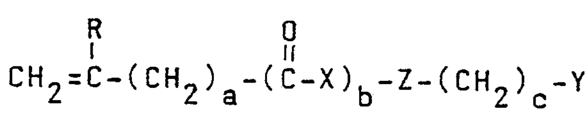

Formule II

dans laquelle
$R = H$ ou $CH_3$
$X = -NH$ ou $-O$
$Y = SO_3^-$ ou $COO^-$
$Z = -(CH_2)_k-^{+}N[(CH_2)_m-H]_2-$, avec $k = 1-12$
$m = 1-6$

ou

Z = 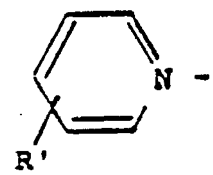

R' représentant H ou un groupe alkyle en $C_1$-$C_3$
a = 0-6
b = 0-1
c = 1-12.

**2.** Procédé selon la revendication 1, caractérisé en ce que le mélange de monomères contient, en tant que composé de formule II, une N-(oméga-sulfoalkyl)-N-acrylamidoalkyl-N,N-dialkylammoniumbétaïne, une N-(oméga-sulfoalkyl)-N-méthacrylamidoalkyl-N,N-dialkylammoniumbétaïne ou les composés N-(oméga-carboxyalkyle) correspondants et on utilise en tant que composé de formule I un acryl- ou méthacrylamidoalkylaldéhyde-dialkylacétaldont les groupes alkyle sont en $C_1$ à $C_8$.

**3.** Procédé selon la revendication 1, caractérisé en ce que le mélange de monomères contient en tant que composé de formule II la N-(3-sulfopropyl)-N-méthacryloxyéthyl-N,N-diméthylammoniumbétaïne, la N-(3-sulfopropyl)-N-méthacrylamidopropyl-N,N-diméthylammoniumbétaïne ou les composés acryliques correspondants, et on utilise en tant que composé de formule I un acryl- ou méthacrylamidoalkyl-aldéhyde-di-n-pentylacétal.

**4.** Procédé selon la revendication 1, caractérisé en ce que le mélange de monomères contient en tant que composé II la 1-(3-sulfopropyl)-2-vinyl-pyridiniumbétaïne, et on utilise en tant que composé de formule I un acryl- ou méthacrylamidoalkyl-aldéhyde-di-n-pentylacétal.

**5.** Procédé selon la revendication 1, caractérisé en ce que le mélange de monomères contient en outre jusqu'à 50 % en poids, par rapport au mélange total, de styrène, vinylnaphtalène ou vinyltoluène.

**6.** Procédé selon la revendication 1, caractérisé en ce que le mélange de monomères contient en outre jusqu'à 30 % en poids, par rapport au mélange total, d'acide méthacrylique, acide acrylique ou acide crotonique.

**7.** Procédé selon la revendication 1, caractérisé en ce qu'en plus des monomères de formule I et II ainsi que de composés choisis parmi le styrène, le vinylnaphtalène et le vinyltoluène et l'un des composés acide méthacrylique, acide acrylique ou acide crotonique, le mélange de monomères contient jusqu'à 90 % en poids, par rapport au mélange total, de diméthylformamide.

**8.** Procédé selon la revendication 1, caractérisé en ce qu'en plus des monomères de formule I et II ainsi que de composés choisis parmi le styrène, le vinylnaphtalène et le vinyltoluène et l'un des composés acide méthacrylique, acide acrylique ou acide crotonique, le mélange de monomères contient jusqu'à 90 % en poids, par rapport au mélange total, d'une solution aqueuse d'émulsifiant à jusqu'à 2 %.

**9.** Procédé pour la préparation de polymères en dispersin biologiquement actifs, caractérisé en ce que l'on prépare des polymères en dispersion selon la revendication 1 et on lie à ceux-ci des anticorps, antigènes ou haptènes.

**10.** Procédé pour la détermination d'antigènes, anticorps ou haptènes, caractérisé en ce que l'on utilise des polymères en dispersion biologiquement actifs, préparés selon la revendication 9.

**11.** Procédé selon la revendication 10, caractérisé en ce que l'on utilise pour la détermination des procédés néphélométriques ou turbidimétriques ou des procédés de comptage de particules.

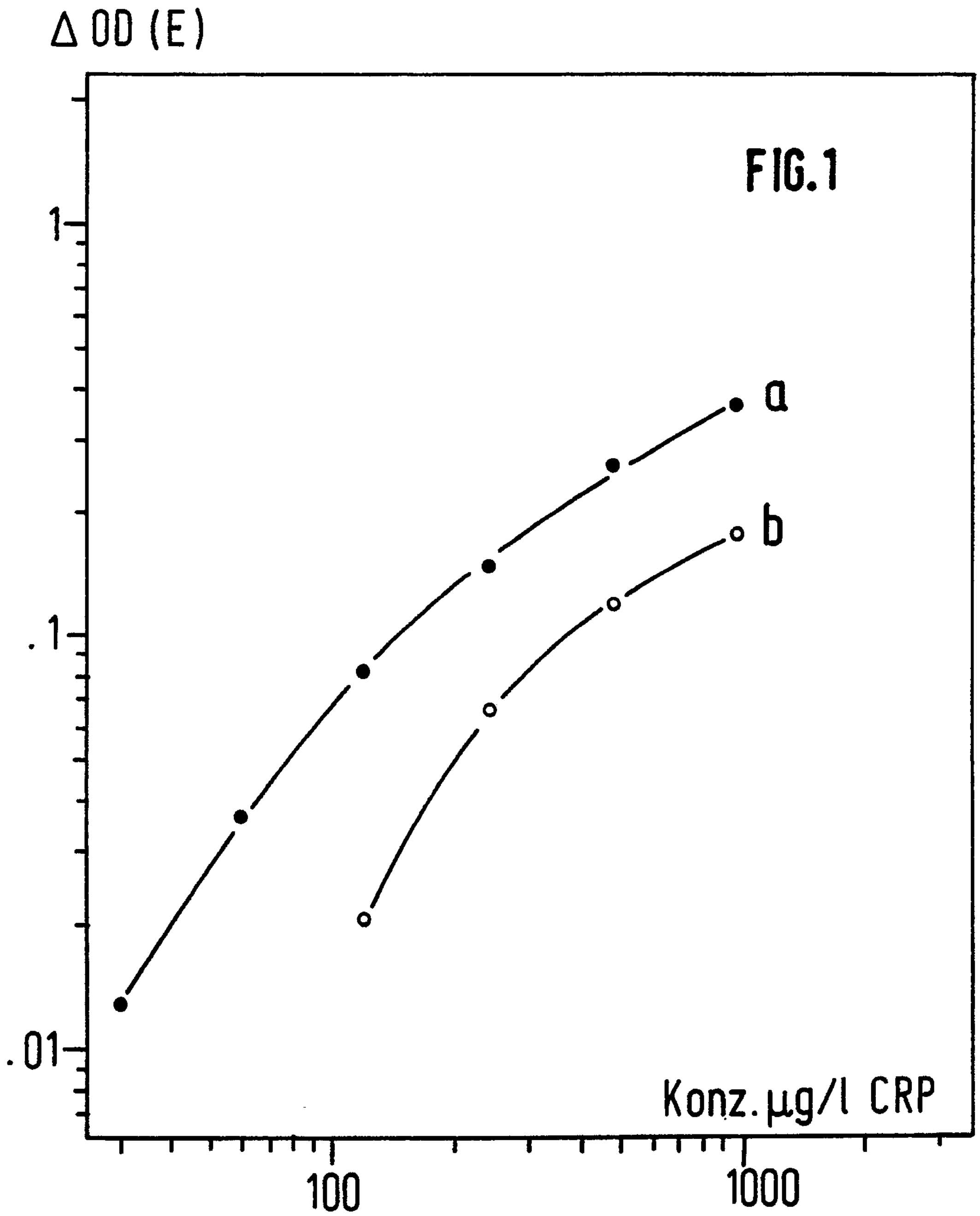


FIG.1

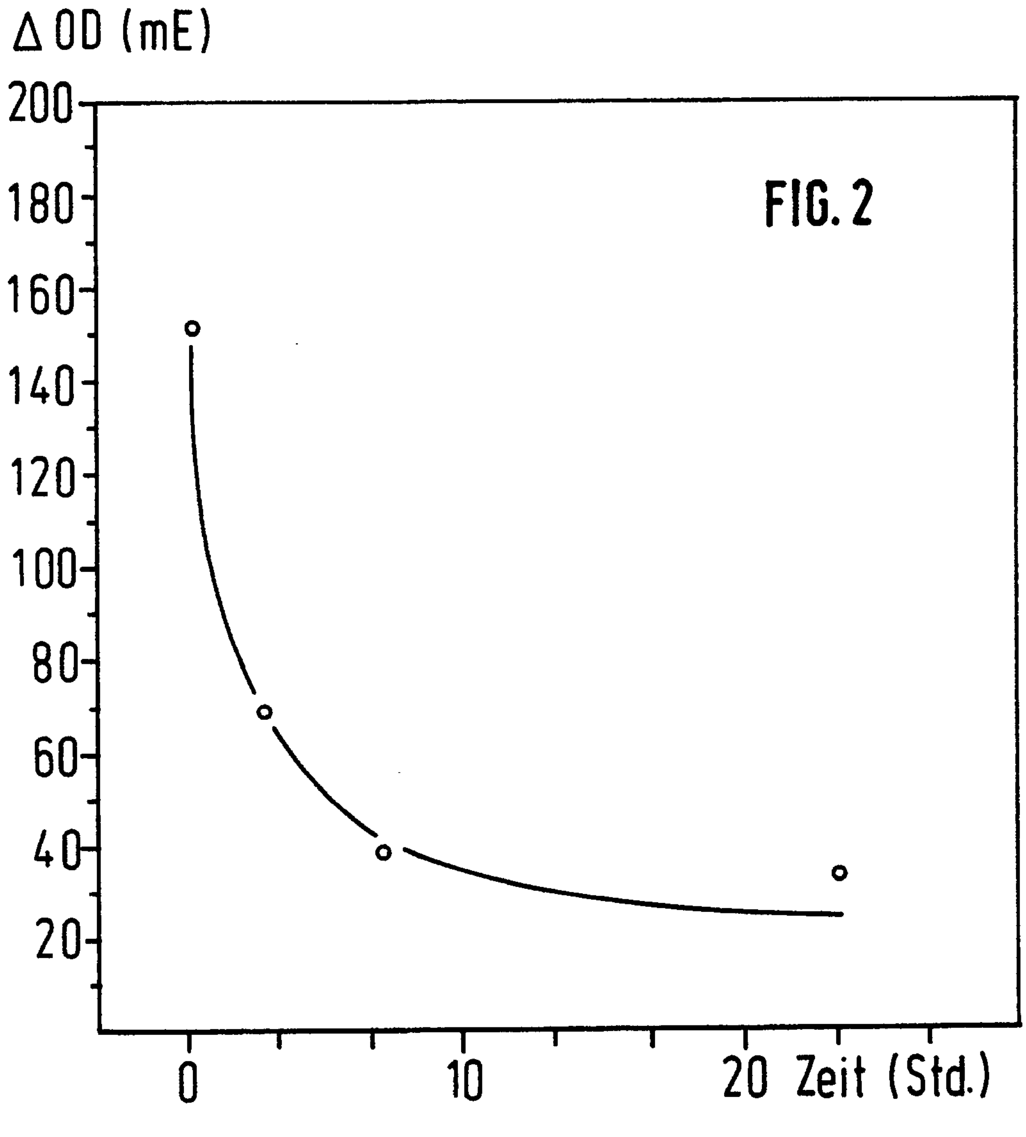


FIG. 2

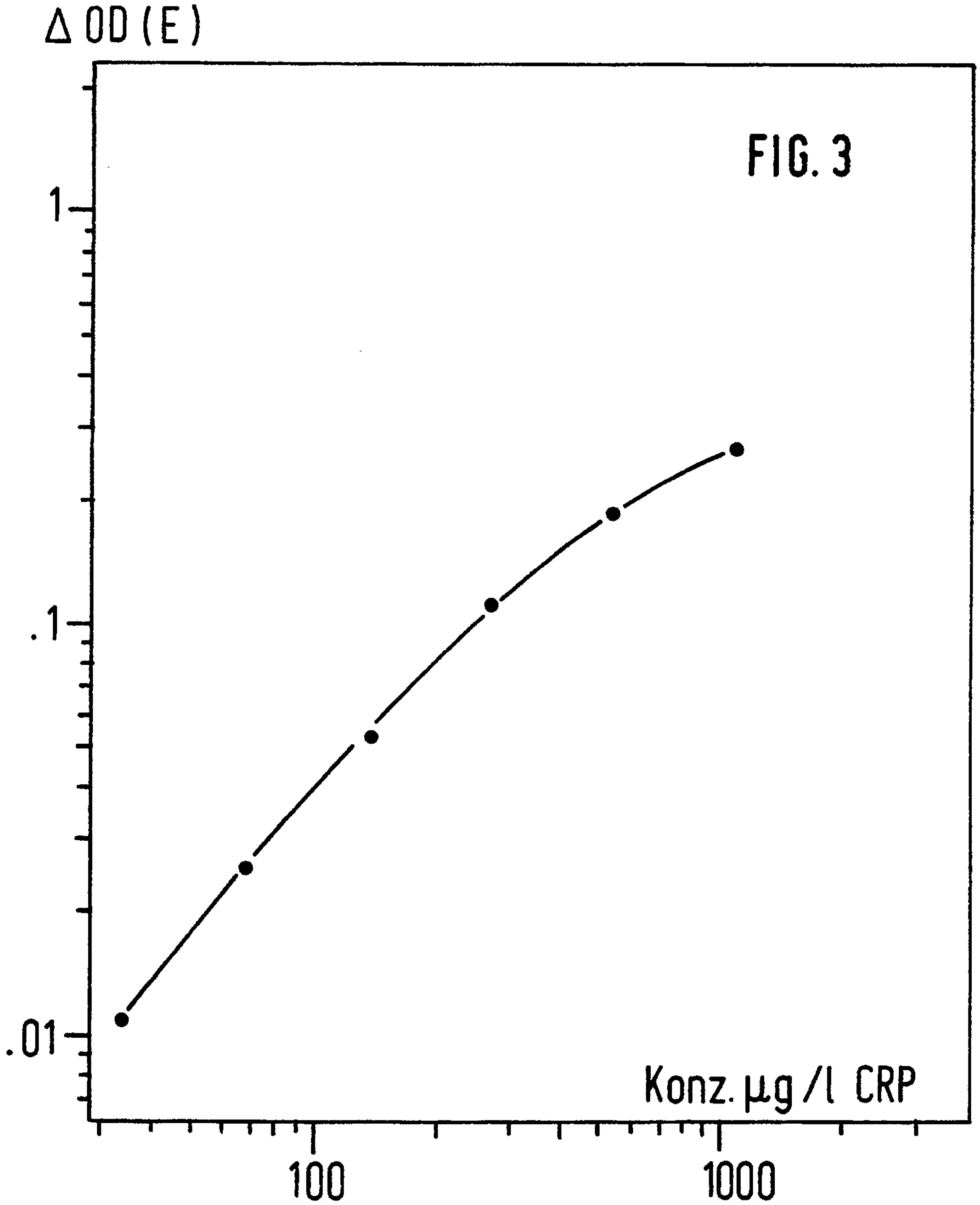


FIG. 3

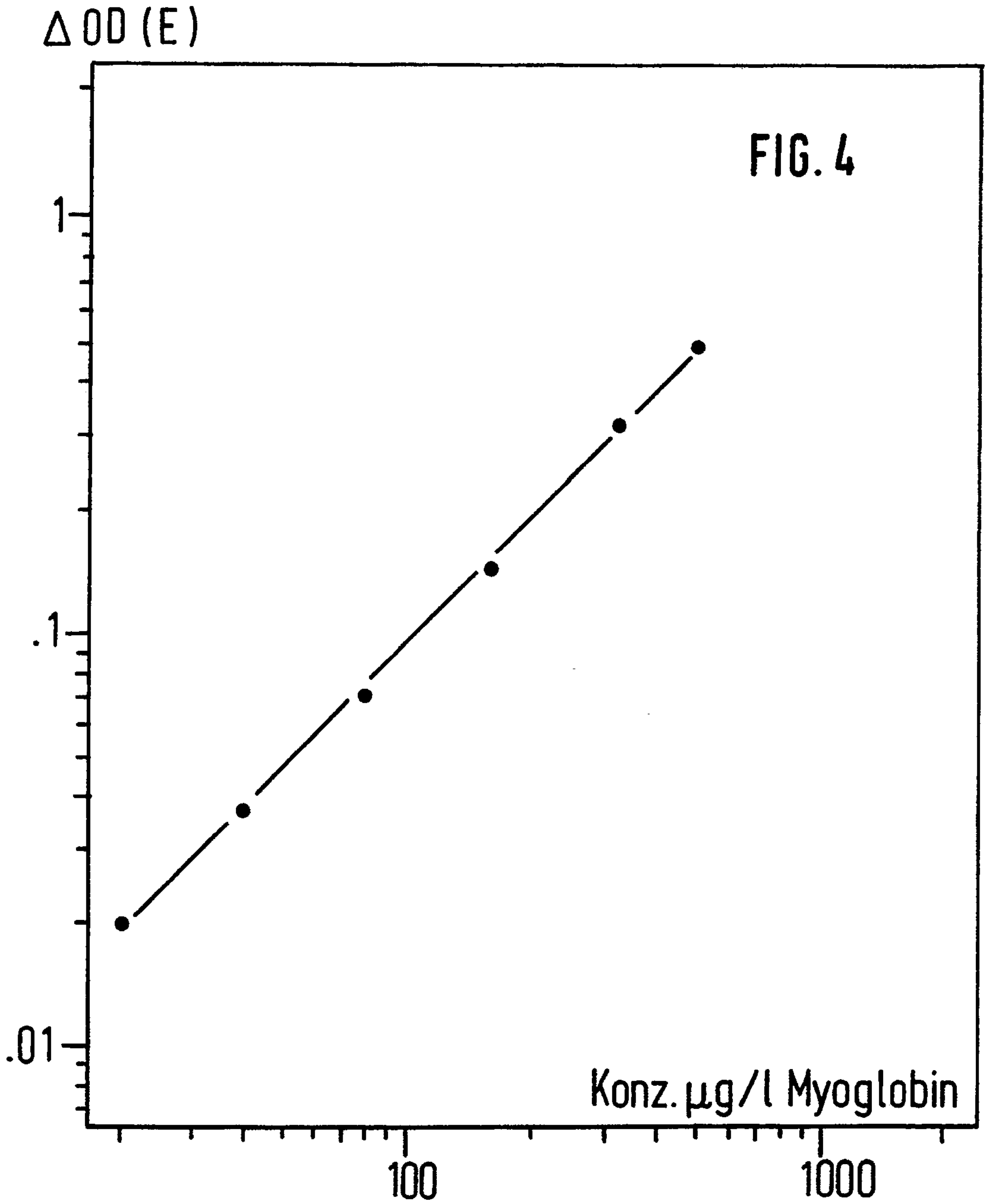
Δ OD (E)
FIG. 4
1
.1
.01
Konz. µg/l Myoglobin
100
1000